# EUROPEAN PATENT SPECIFICATION

(11) **EP 0 512 071 B1**
(45) Date of publication and mention of the grant of the patent: **30.10.1996**
(21) Application number: 91904851.2
(22) Date of filing: 25.01.1991
(51) Int. Cl.: A61K 38/00, A61K 39/00, C07K 2/00, C12N 5/00

(54) **METHOD AND COMPOSITIONS FOR INHIBITING ANGIOGENESIS**
VERFAHREN UND ZUSAMMENSETZUNGEN ZUR HEMMUNG DER ANGIOGENESE
PROCEDE ET COMPOSITIONS DESTINES A INHIBER L'ANGIOGENESE

(30) Priority: 25.01.1990 US 470067; 02.10.1990 US 591559
(43) Date of publication of application: 11.11.1992
(73) Proprietor: CHILDREN'S HOSPITAL, Boston, MA 02115 (US)
(72) Inventor: MOSES, Marsha, A., Brookline, MA 02146 (US); SUDHALTER, Judith, Newton Lower Falls, MA 02162 (US)
(74) Representative: Bassett, Richard Simon
(86) International application number: US9100559
(87) International publication number: WO9111193

(56) References cited:
- WO-A-90/12590
- US-A- 4 743 587
- US-A- 4 766 111
- SCIENCE, vol. 248, 15 June 1990, LANCASTER, PA, US; pages 1408-1410; M.A. MOSES ET AL.: 'Identification of an Inhibitor of Neovascularization from Cartilage'
- BIOLOGICAL ABSTRACTS, vol. 83, no. 12, 1987, Philadelphia, PA, US; abstract no. 119447; M. TAKIGAWA ET AL.: 'A factor in conditioned medium of rabbit costal chondrocytes inhibits the proliferation of cultured endothlial cells and angiogenesis induced by b16 melanoma: Its relation with cartilage-derived anti-tumor factor (CATF)
- JOURNAL OF BIOLOGICAL CHEMISTRY. (MICROFILMS), vol. 264, no. 29, 15 October 1989, BALTIMORE, MD, US, pages 17445-17453; Y.A. DE CLERCK ET AL.: 'Purification and Characterization of Two Related but Distinct Metalloproteinase Inhibitors Secreted by Bovine Aortic Endothelial Cells'
- NATURE, vol. 318, issued 07 November 1985, pages 66-69; DOCHERTY et al.: "Sequence of Human Tissue Inhibitor of Metalloproteinases and its Identity of Erythroid Potentiating Activity", pages 66-69, see page 66.
- BIOCHIMICA ET BIOPHYSICA ACTA, vol. 924, issued 1987, pages 238-247; GABRIELIDES et al.: "A Vertebrate Interstitial Collagenase Inhibitor from Bovine Scapular Cartilage: Parification and Characterization"
- SCIENCE, vol. 221, issued 19 August 1983, pages 719-725; FOLKMAN et al.: "Angiogenesis Ihnibition and Tumor Regression Caused by Hepanin or a Hepanin Fragment in the Presence of Cortisone"
- SCIENCE, vol. 221, issued 16 September 1983, pages 1185-1187; LEE et al.: "Shark Cartilage Contains Inhibitors of Tumor Angiogenesis"
- JOURNAL OF IMMUNOLOGICAL METHODS, vol. 33, issued 1980, pages 239-247; FALK et al.: "A 48-Well Micro Chemotaxis Assembly for Rapid and Accurate Measurement of Leucocyte Migration"
- THE JOURNAL OF CELL BIOLOGY, vol. 107, no. 6, part 3, issued December 1988, Moses et al., "Bovine Scapalan Cartilage Contains an Inhibitor of Capillary Endothelial Cell Migration", page 374a, see abstract 2132.
- BIOLOGICAL ABSTRACTS, vol. 85, no. 11, issued 01 June 1988, page 115, column 1, abstract no. 107968, Collagen Relat. Res., 8(1), 1-10; CRESPO et al.: "Monoclonal Antibodies Against Synovial Collagenase: Use for Immunopurification and Characterization of the Latent and Active Enzyme"

## Description

### Background of the Invention

The United States government has rights in this invention by virtue of grants from the National Institutes of Health.

Angiogenesis, the process of new capillary formation, plays an important role in numerous physiological events, both normal and pathological. Angiogenesis is a complex process characterized by infiltration of basement membrane by capillary endothelial cells, the migration of cells through matrix towards a stimulus, and subsequent cell proliferation, resulting in new capillary tube formation.

A number of proteases have been implicated as key factors in angiogenesis, but no one enzyme has been demonstrated to be critical in all three components of angiogenesis: endothelial cell migration and proliferation and capillary tube ingrowth. See, for example, Mignatti, et al., Cell 47, 487-498 (1986) and Rifkin, et al., Acta. Biol. Med. Germ. 40, 1259-1263 (1981), who suggest that a number of enzymes in a proteolytic cascade, including plasminogen activator and collagenase, must be inhibited in order to inhibit angiogenesis. Plasminogen activator and collagenase interact via plasmin, which is cleaved from plasminogen by plasminogen activator, and cleaves the latent form of collagenase to yield the active form. As a result, no one enzyme inhibitor has been shown to be able to inhibit angiogenesis in the absence of cytotoxicity.

Under normal conditions, angiogenesis is associated with such events as wound healing, corpus luteum formation and embryonic development, as discussed by Folkman, et al., Science 243, 1490-1493 (1989). However, a number of serious diseases are also dominated by abnormal neovascularization including solid tumor growth and metastases, some types of eye disorders, and rheumatoid arthritis, reviewed by Auerbach, et al., J. Microvasc. Res. 29, 401-411 (1985); Folkman, Advances in Cancer Research, eds. Klein and Weinhouse, pp. 175-203 (Academic Press, New York 1985); Patz, Am. J. Opthalmol. 94, 715-743 (1982); and Folkman, et al., Science 221, 719-725 (1983). For example, there are a number of eye diseases, many of which lead to blindness, in which ocular neovascularization occurs in response to the diseased state. These ocular disorders include diabetic retinopathy, neovascular glaucoma, inflammatory diseases and ocular tumors (e.g. retinoblastoma). There are a number of other eye diseases which are also associated with neovascularization, including retrolental fibroplasia, uveitis, approximately twenty eye diseases associated with choroidal neovascularization and approximately forty eye diseases which are associated with iris neovascularization.

The current treatment of these diseases is inadequate, especially once neovascularization has occurred, and blindness often results. Studies have suggested that vaso-inhibitory factors which are present in normal ocular tissue (cornea and vitreous) are lost in the diseased state. The potential therapeutic benefit that a naturally occurring inhibitor of angiogenesis might have in controlling diseases in which neovascularization plays a critical role has prompted a long term search for angiogenesis inhibitors. An inhibitor of angiogenesis could have an important therapeutic role in relieving the course of these disorders, as well as provide a valuable means of studying their etiology.

A number of investigators have reported that extracts of cartilage, one of the few avascular tissues in the body, can inhibit angiogenesis: Eisentein, et al., Am. J. Pathol. 81, 1-9 (1987); Pauli, et al., J. Natl. Cancer Inst. 67,55-74 (1981); Brem and Folkman, J. Exp. Med. 141, 427-439 (1975); Langer, et al., Science 193, 70-72 (1976); Langer, et al., Proc. Natl. Acad. Sci. USA 77, 431-435 (1980); and Lee and Langer, Science 221, 1185-1187 (1983). Langer, et al., showed that cartilage extracts containing a collagenase inhibitor retard tumor-induced and inflammatory-induced neovascularization in the cornea and conjunctiva, when delivered by either infusion or sustained release from a polymeric implant.

Other inhibitors of collagenase in vitro have not inhibited angiogenesis in in vivo testing, including α₂-macroglobulin and tissue inhibitor metalloproteinase (TIMP). Angiogenesis has several key components, including proliferation of capillary endothelial cells, migration of endothelial cells, and the production of collagenase. To inhibit angiogenesis, a compound, alone or in combination with one or more other factors, must inhibit proliferation of capillary endothelial cells, migration of endothelial cells, and collagenase. At the dosages tested, TIMP inhibits capillary endothelial cell proliferation but not angiogenesis, and protamine inhibits blood vessel ingrowth but not proliferation of capillary endothelial cells. Protamine was cytotoxic at concentrations appearing to inhibit angiogenesis.

Extracts from several other tissue sources have also been shown to have anti-angiogenic activity, as reviewed by D'Amore, Prog. Clin. Biol. Res. 221, 269-283 (1986). Several molecules have been identified which inhibit different aspects of angiogenesis, such as cell proliferation or cell migration, although no single molecule capable of inhibiting angiogenesis in vivo has been identified in the prior art until reported by applicants in Science 248, 1408-1410 (1990), as described in U.S. Serial No. 07/027,364 filed March 18, 1987, Langer, et al. This application, which is a continuation-in-part of earlier applications reporting on a partially purified protein extract isolated from cartilage having anti-angiogenic activity, discloses the purification of a single protein that inhibits collagenase, and is also thought to be an inhibitor of angiogenesis, tumor-induced vascularization, connective tissue breakdown, and tumor cell invasion, and therefore useful in the treatment of corneal ulcers and rheumatoid arthritis and psoriasis, based on the inhibition of collagenase.

It is an object of the present invention to provide pharmaceutical compositions, and method of use thereof, for the treatment of diseases involving abnormal angiogenesis.

It is a further object of the present invention to provide pharmaceutical compositions, and method of use thereof, for inhibition of capillary endothelial cell proliferation and migration.

It is a still further object of the present invention to provide pharmaceutical compositions, and method of use thereof, which inhibit capillary endothelial cell proliferation and migration, as well as collagenase, or activation of collagenase, at pharmaceutically useful dosages.

It is another object of the present invention to provide topical and controlled release pharmaceutical compositions, and methods of use thereof, for inhibition of angiogenesis.

### Summary of the Invention

An isolated and purified protein wherein the protein: a) is a normal component of cartilage; b) has an apparent molecular weight by SDS-PAGE under reducing conditions of about 34 kD when isolated without exposure to acid pH or of about 28 kD after exposure to pH 2.0; c) is a collagenase inhibitor; and d) produces an avascular zone in a chorioallantoic membrane assay when used in the amount of about 4 µg.

The protein may be used for inhibition of blood vessel growth in vivo and capillary endothelial cell proliferation and migration in vivo. The protein can be extracted from bovine scapular cartilage using 2 M NaCl, followed by a series of precipitation and column chromatography steps; or expressed from nucleotide sequences encoding the protein, isolated using probes based on the NH₂-terminal protein sequence.

The effective dosage for inhibition of angiogenesis in vivo, defined as inhibition of capillary endothelial cell proliferation and migration and blood vessel ingrowth, is extrapolated from in vitro inhibition assays. Effective dosages in vitro range from 1 nM, for inhibition of capillary endothelial cell migration to 100 nM, for inhibition of capillary endothelial cell proliferation. The preferred dosage in vivo is between 16 nM and 50 to 75 nM. The effective dosage is dependent on the method and means of delivery, which can be localized or systemic. For example, in some applications, as in the treatment of psoriasis or diabetic retinopathy, the inhibitor is delivered in a topical ophthalmic carrier. In other applications, as in the treatment of solid tumors, the inhibitor is delivered by means of a biodegradable, polymeric implant.

In vitro assays have been developed to screen for compounds effective as angiogenesis inhibitors in vivo. These assays represent a key event required for angiogenesis, so that inhibition of the assay is representative of inhibition of angiogenesis in vivo. Events that are tested include proteolytic degradation of extracellular matrix and/or basement membrane; proliferation of endothelial cells, migration of endothelial cells, and capillary tube formation.

### Detailed Description of the Invention

It has now been discovered, in contrast to numerous reports in the literature, that all of the different important factors of angiogenesis: proliferation and migration of endothelial cells, tube formation and ingrowth of blood vessels, can be inhibited solely by specifically inhibiting collagenase. This is most surprising in view of the many different pathways and enzymes involved in angiogenesis. It was not expected to find a common element of enough significance to allow specific inhibition of angiogenesis in the absence of cytotoxicity.

### In vitro Assays for determining effectiveness and effective concentration of angiogenesis inhibitors in vivo.

Assays are used to screen compounds for inhibition of collagenase activity, proteolytic degradation of extracellular matrix or basement membrane, migration of endothelial cells, and capillary tube formation to determine (1) potential effectiveness in vivo as inhibitors of angiogenesis and (2) the effective concentration. As discussed above, all of these elements of angiogenesis must be inhibited in vivo for a composition to be effective. It has been determined that collagenase inhibitors are effective in all of these assays, if provided in a suitable carrier and concentration. The assays are therefore first used to determine if the compound is a collagenase inhibitor, then if it is active in the other assays and at what dosage.

The essential elements of these assays include using endothelial cells as the assay target and stimulation of the endothelial cells with known angiogenesis factors to compare a putative inhibitor's effect in unstimulated, as compared to stimulated, endothelial cells. Endothelial cells that can be used include capillary endothelial cells and umbilical vein endothelial cells. Factors which can be used to stimulate the endothelial cells include acidic or basic fibroblast growth factor.

The following assays are based on assays described in the prior art that have been modified for the purpose of screening collagenase inhibitors for inhibition of angiogenesis in vivo.

### Assay for Inhibition of Collagenase Activity.

A unit of collagenase activity is defined as that amount of protein required to inhibit one unit of corneal collagenase by 50%. One unit of collagenase produces 10% cleavage of collagen in 2.5 h at 37°C.The collagenase activity is measured using an assay developed by Johnson-Wint, Anal. Biochem. 68, 70 (1980). Type I collagen is purified from rat tail tendon by solubilization in 0.5 M acetic acid and repeatedly precipitated with sodium phosphate, and then acetylated using [¹⁴C] acetic anhydride. The radiolabelled collagen is dissolved in 0.5 M acetic acid together with sufficient non-labelled purified Type I collagen to make a solution of 2 mg/ml with a specific activity of approximately 350,000 cpm/ml. The collagen solution is dialyzed against two changes of 0.15 M sodium phosphate buffer, pH 7.6, at 4°C. Ninety-six well tissue culture plates are placed at a 45° angle and aliquots of the dialyzed solution (25 µl, 8,750 cpm) are added to the lower half of each well. Fibrils are allowed to form by incubating the plates for 1 h at 37°C. Plates are immersed in water for 1 h, then allowed to dry overnight.

Collagenase is obtained from the culture medium of explanted bovine corneas. Corneas are excised from bovine eyes and rinsed in clinidine solution and then Hanks' balanced salt solution containing 2% penicillin-streptomycin. They are then cut into 4 mm² pieces and placed in T150 tissue culture flasks containing Dulbecco's modified Eagle's medium, 5% fetal bovine serum, and 2% penicillin-streptomycin solution. 0.5 ml of cytochalasin B (1 mg/ml in dimethyl sulfoxide) is then added to 100 ml of medium. One flask contains approximately 10 corneas/50 ml of medium. The flasks are incubated at 35°C in an incubator with humidified 95% air, 5% CO₂. The medium is collected 7 days after corneas are explanted and stored at 4°C. Fresh medium is added to the corneas and a second collection made at day 14. Collagenase-containing medium is stored under sterile conditions for up to two months at 4°C. Before use, latent collagenase present in the culture medium is activated by trypsin treatment, by incubating the medium for seven min at 37°C with one-tenth volume of 1 mg/ml of trypsin. Following activation, one-tenth volume of soybean trypsin inhibitor (5 mg/ml) is added to quench the trypsin. The enzyme is then diluted with collagenase assay buffer (CAB)(0.05 M Tris-HCl, pH 7.6, containing 0.2 M NaCl, 0.001 M CaCl₂, and 0.01% sodium azide, CAB) such that 100 µl collagenase solution produces cleavage of 10% (5 µg) collagen in 2.5 h at 37°C.

Samples to be tested for collagenase inhibitory activity are dissolved in, or dialyzed against, CAB. 100 µl samples and 100 µl of diluted enzyme solution are mixed together, added to collagen-containing wells, and incubated for 2.5 h at 37°C. The supernatants, containing soluble radioactively labelled degradation products, are counted in a Beckman Model LS 1800 scintillation spectrometer.

### Assay for Inhibition of capillary endothelial cell proliferation.

Capillary EC proliferation in response to an angiogenic stimulus is a critical component of neovascularization, as discussed by Ausprunk and Folkman, J. Microvasc. Res. 14, 153-65 (1977). By utilizing the specific cells involved in angiogenesis, and stimulating them with known angiogenesis factors, in this case acidic fibroblast growth factor (aFGF), as reported by D'Amore and Klagsbrun, J. Cell Biol. 99, 1545-1549 (1984), it is possible to mimic the angiogenesis process in vitro. This type of assay is the assay of choice to demonstrate the stimulation of capillary EC proliferation by various angiogenic factors, as reviewed by Shing, et al., Science 223, 1296-1298 (1984).

The number of endothelial cells in culture can be quickly determined based on the colorimetric determination of cellular acid phosphatase activity, described by Connally, et al., J. Anal. biochem. 152, 136-140 (1986). Capillary endothelial cells are plated (2 x 10³ /0.2 ml) onto gelatin-coated 96-well tissue culture dishes on day 1. On day 2, cells are refed with Dulbecco's modified Eagle's medium (Gibco) with 5% calf serum (Hyclone) (DMEM/5) and aFGF (2 ng/ml) (FGF Co.) and increasing concentrations of compound added. The compound is added in volumes that do not exceed 10% of the final volume. Wells containing phosphate buffered saline (PBS) (Gibco) alone and PBS + aFGF are included as controls. On day 5, media are removed and cells are washed with PBS and lysed in 100 µl of buffer containing 0.1 M sodium acetate (pH 5.5), 0.1% Triton X-100™ and 100 mM p-nitrophenyl phosphate (Sigma 104 phosphatase substrate). After incubation for 2 hours at 37°C, the reaction is stopped with the addition of 10 µl of 1 N NaOH. Color development is determined at 405 nm using a rapid microplate reader (Bio-Tek).

According to Connally, et al., a linear relationship is obtained between acid phosphatase activity and endothelial cell number up to 10,000 cells/well. Standard curves for acid phosphatase activity are also generated from known cell numbers in order to verify that the enzyme levels reflect the actual EC numbers. Percent inhibition is determined by comparing the cell number of wells exposed to stimulus with those exposed to stimulus and inhibitor. Each point represents the mean of quadruplicate control wells and triplicate inhibitor wells and the SEM is less than 7% of the mean. Electronic cell counting assays confirm the accuracy of the data.

The effect of the compound on capillary EC synthesis of DNA, can also be determined by the suppression of the incorporation of radioactive thymidine by capillary EC in response to aFGF.

### Assay for Inhibition of capillary Endothelial Cell Migration.

A modification of the Boyden chamber technique, Falk, et al., J. Immunol. 118, 239-247 (1980), is used to study the effect of CDI on capillary EC migration. A blind-well Boyden chamber, as described in J. Exp. Med. 115, 453-456 (1962), consists of two wells (upper and lower) separated by a porous membrane. A known concentration of growth factor is placed in the lower wells and a predetermined number of cells and compound to be tested are placed in the upper wells. Cells attach to the upper surface of the membrane, migrate through and attach to the lower membrane surface. The membrane can then be fixed and stained for counting, using the method of Glaser, et al., in Nature 288, 483-484 (1980).

Migration is measured using blind well chambers (Neuroprobe, no. 025-187) and polycarbonate membranes with 8-µm pores (Nucleopore) pre-coated with fibronectin (6.67 µg/ml in PBS) (human, Cooper). Basic FGF (Takeda Co.) diluted in DMEM with 1% calf serum (DMEM/l) is added to the lower well at a concentration of 10 ng/ml. The upper wells receive 5 x 10⁵ capillary EC/ml and increasing concentrations of compound to be tested. Control wells receive DMEM/l, either with or without bFGF. The migration chambers are incubated at 37°C in 10% CO₂ for 4 hours. The cells on the upper surface of the membrane are then wiped off by drawing the membrane over a wiper blade (Neuroprobe). The cells which have migrated through the membrane onto the lower surface were fixed in 2% glutaraldehyde followed by methanol (4°C) and stained and hematoxylin. Migration is quantified by counting the number of cells on the lower surface in 16 oil immersion fields (OIF). Each point represents the mean +/- SEM of 4 wells. In control wells without bFGF, the number of migrated capillary ECs is 61 +/- 7 cells.

### Assay for Inhibition of Neovascularization in vitro.

The chick chorioallantoic membrane assay (CAM), described by Taylor and Folkman, Nature (London) 297, 307-312 (1982), is used to determine whether the compound is capable of inhibiting neovascularization in vivo. The effect of the compound on growing embryonic vessels is studied using check embryos in which capillaries appear in the yolk sac at 48 h and grow rapidly over the next 6-8 days.

On day 3 of development, fertilized chick embryos are removed from their shells and placed in plastic petri dishes (1005, Falcon). They are maintained in humidified 5% CO₂ at 37°C. On day 6, 4 µg samples of purified CDI are mixed in methylcellulose disks and applied to the surfaces of the growing CAMs above the dense subectodermal plexus. Following a 48 hour exposure of the CAMs to CDI, avascular zones, free of capillaries and small vessels are observed using a binocular dissecting microscope at x7⁻¹⁰ magnification. The CAMs are injected intravascularly with India ink/Liposyn as described previously by Taylor and Folkman (1982).

Tissue specimens are fixed in formalin at room temperature and rinsed in 0.1 M cacodylate buffer pH 7.4. The specimens are embedded in JB-4 plastic (Polysciences) at 4°C and 3 µm sections are cut using a Reichert 2050 microtome. Sections are stained with toluidine blue and micrographs taken on a Zeiss photomicroscope using Kodak TM x100 and a green filter.

### Assay for Inhibition of Capillary Tube Formation.

Capillary tube formation is an important component of angiogenesis. An assay which is indicative of capillary tube formation activity consists of applying the compound to, or between, collagen gels and determining if capillary-like tubes are formed by cultured BCE cells. In control collagen gels, not treated with inhibitor, a monolayer of BCE cells appears as a network of branching, anastomosing cords of endothelial cells resembling capillary beds in vivo. In contrast, collagen gels treated with an inhibitor show major disruption of the BCE cells reorganization such that any organization into capillary-like tubes appears to be prevented.

### Examples of determination of in vivo anti-angiogenic activity of cartilage derived inhibitor based on in vitro assays.

Purified protein derived from cartilage (CDI) was isolated and characterized as having at least three distinct activities important in inhibition of angiogenesis. On the basis of these activities, the protein can be delivered in a pharmaceutically acceptable carrier in a dosage inhibiting angiogenesis in vivo, by locally inhibiting capillary endothelial cell proliferation and migration and blood vessel ingrowth.

As used herein, CDI refers to any protein specifically inhibiting eukaryotic collagenase encoded by a nucleotide sequence hybridizing under standard conditions to the nucleotide sequence encoding the inhibitor protein isolated from cartilage, as described below, or from chondrocyte or fibroblast culture fluid. Because CDI is not species specific, it is possible to use CDI isolated from non-human sources to treat humans. CDI-like proteins that specifically inhibit collagenase are produced by a number of different tissues. As a result, CDI proteins can also be expressed from genetically engineered sequences isolated by standard techniques from a library prepared from chondrocytes or other cells such as fibroblasts which can differentiate into cartilage, or other mammalian cells, using labeled synthetic oligonucleotides encoding portions of the CDI isolated from cartilage as probes. These probes can be synthesized using commercially available equipment or by a company that performs synthesis on a contract basis. Methods for screening libraries using labelled probes or antibodies to a purified protein are well known to those skilled in the art, as taught, for example, by Maniatis, et al., Molecular Cloning: A Laboratory Manual (Cold Spring Harbor, NY 1989).

Partial purification and characterization of the collagenase inhibition activity of the protein are described in detail in U.S. Serial No. 07/027,364 filed March 18, 1987. The cartilage-derived inhibitor (CDI) is isolated from bovine cartilage via a 2 M NaCl extraction followed by precipitation with hydrochloric acid and two ammonium sulfate precipitations (25%, 60%). The inhibitor is applied to an A-1.5 m gel filtration column. Further purification is achieved by ion exchange chromatography, a second gel filtration step and reverse-phase high performance liquid chromatography.

CDI can be isolated from cartilage. Veal scapulae are obtained from a slaughterhouse within 24 h after slaughter. The half of the cartilage furthermost from the bone is excised and scraped first with a periosteal elevator (Arista) and then with a scalpel blade (No. 10, Bard-Parker) until clean of connective tissue. Cartilage slices are stored at - 20°C until needed.

Frozen cartilage is thawed and chopped to form pieces of 1/8 in³ in a Cuisinart Model DLC 10. Chopped slices (1 kg per batch) are extracted in 8 L. 0.01 M 4-(2-hydroxyethyl)-1-piperazineethanesulfonic acid, pH 7.6, containing 2 M NaCl, 3 mM EDTA, and 0.02% sodium azide for 48 h at 4°C. The extract is filtered through a Nitex filter (153 µ mesh, Tetko), centrifuged at 12,500 x g for 1 h at 4°C and then filtered through both Nitex and multiple layers of cheesecloth. The filtered extract, approximately 7.4 L, is concentrated to 500 ml using a Pellicon Cassette System (Millipore) equipped with one PTCG cassette (molecular weight cutoff, Mᵣ 10,000). The concentrate is dialyzed against 10 mM Tris-HCl, pH 7.6 containing 0.15 NaCl and 0.02% sodium azide, and store at -20°C.

To acid precipitate contaminants of the inhibitor, 1 N HCl is added dropwise to the concentrated extract until the pH reaches 2.0. The extract is centrifuged at 25,000 x g for 30 min and the pellet discarded. 1 N NaOH is added dropwise to the supernatant until the pH is 7.6. The extract is centrifuged at 25,000 x g for 30 min and the pellet discarded to remove any acid insoluble contaminants from the inhibitor-containing solution.

(NH₄)₂SO₄ is added to the extract to 25% saturation and the precipitate discarded after centrifugation. More (NH₄)₂SO₄ is added until 60% saturation is reached. the 60% saturation solution is centrifuged and the inhibitor-containing pellet stored at -20°C.

The thawed pellet is dissolved in 0.01 M Tris-HCl, pH 7.6 containing 4 M guanidine-HCl, 0.001 M CaCl₂, and 0.02% sodium azide, and applied to an A1.5m Sepharose™ size exclusion column (Pharmacia, Piscataway, NJ) (2.5 x 90 cm) at a flow rate of 30 ml/h, 4°C. Column effluents are monitored spectrophotometrically at 280 or 220 nm. The inhibitor elutes at an apparent molecular weight of 28,000 Da. All fractions containing inhibitor by assay of collagenase activity are pooled and concentrated in an Amicon-stirred cell equipped with a YM10 membrane. The retentate is dialyzed against 0.05 M Tris-HCl, pH 7.6 containing 0.05 M CaCl₂, and 0.05% sodium azide, then applied to a Bio-Rex 70 ™ cation exchange column (200-400 mesh, 2.6 x 7.5 cm) at a flow rate of 70 ml/h. The column is washed with ten column volumes of 0.05 M Tris-HCl, pH 7.6 containing 0.05 M CaCl₂, and 0.05% sodium azide to elute nonabsorbing protein. Bound material is eluted with a salt gradient, 0.05 to 0.75 M NaCl in 0.05 M Tris-HCl, pH 7.6, containing 0.001 M CaCl₂ and 0.01% sodium azide. The inhibitor binds to the column in low salt, 0.05 M NaCl, and eluted up to about 0.2 M NaCl.

Fractions containing inhibitor are concentrated in the stirred cell and dialyzed against collagenase assay buffer, CAB, 0.05 M Tris-HCl, pH 7.6, containing 0.2 M NaCl, 0.001 M CaCl₂, and 0.01% sodium azide. The dialysate is applied to a Sephadex G-75 ™ (superfine, Pharmacia) column (1.6 x 50 cm) column, equilibrated with CAB, at a flow rate of 10 ml/h. Fractions containing inhibitor eluted after the dominant peak of an A₂₈₀ absorbing material, and were pooled and concentrated in the stirred cell to a volume of 1 ml. An equal volume of 0.05% trifluoroacetic acid in water is added to the sample. Reverse-phase high performance liquid chromatography is then conducted on a C₄ column. A linear gradient of 0.05% trifluoroacetic acid in water to 0.05% trifluoroacetic acid in acetonitrile is used to elute bound protein. 100 µl aliquots are removed and immediately dialyzed against CAB for assaying collagenase activity.

The collagenase inhibitor is purified to apparent homogeneity by reverse-phase high performance liquid chromatography. Total protein, total units, specific activity recovery, and purification for each step are listed in Table I.

**Table I**

| **Purification of a bovine cartilage-derived collagenase inhibitor.** | | | | | |
|---|---|---|---|---|---|
| Step | Total protein (mg) | Total Units^{a} | Specific activity (units/mg) | Recovery (%) | Purification (x fold) |
| concentrated extract | 25,900 | 398,462 | 15.4 | | |
| acid and (NH₄)₂SO₄ ppt. | 1,520 | 84,444 | 55.6 | 21.2 | 3.6 |
| A1.5m | 192 | 42,667 | 222.2 | 10.7 | 14.4 |
| Bio-Rex 70 | 28.5 | 25,000 | 877.2 | 6.3 | 57.0 |
| Sephadex G75 | 4.5 | 12,712 | 2,824.9 | 3.2 | 183.4 |
| RHPLC | 0.15 | 3,000 | 20,000.0 | 0.75 | 1,298.5 |

| | | | | | |
|---|---|---|---|---|---|
| a A unit is defined as that amount of protein required to inhibit one unit of corneal collagenase by 50%. One unit of collagenase produces 10% cleavage of collagen in 2.5 h at 37°C. | | | | | |

CDI-like protein is isolated from conditioned media (Serum free HAM's F12 media supplemented with ascorbic acid, Gibco) in which chondrocytes from bovine scapula have been cultured by exhaustively dialyzing the media against distilled water, then applying the dialysate to a Sephadex G75 gel™ (superfine, Pharmacia) filtration column (1.6 x 50 cm) in 0.05 M Tris-HCl, pH 7.6, containing 0.2 M NaCl, 0.001 M CaCl₂, and 0.01% sodium azide, at a flow rate of 10 ml/h. Fractions were collected and characterized as a single band with a molecule weight of approximately 34,000 by SDS-PAGE under reducing conditions. These fractions inhibited collagenase, as measured above. The fractions were also active in both proliferation and migration assays, as well as in the CAM angiogenesis inhibition assay, with 80% inhibition at 4 micrograms.

Automated Edman degradation of CDI was performed on an Applied Biosystems, Inc. Model 477A protein sequencer using the manufacturer's standard program NORMAL-1. The resulting phenylthio-hydantoin-amino acid fractions were subsequently identified using and on-line (ABI) Model 120A HPLC. A search of the NBRF-PIR protein sequence data base,National Biomedical Research Foundation, Division of Research Resources National Institute of Health, Bethesda, MD, ID# A20595 (1989), revealed that this protein differs from a collagenase inhibitor isolated from human amniotic fluid (which itself is virtually identical to that of a human skin fibroblast inhibitor with the exception of one residue difference), in only two amino acids, at position 17 (valine for leucine) and at position 27 (alanine for proline) for 28 N-terminal residues, reported by Docherty, et al., Nature 318, 66-69 (1985). Samples were not reduced and alkylated, therefore, (---) represent bonafide blanks which align by homology with expected cysteine residues of previously reported collagenase inhibitors; all three residues are in agreement, based on Docherty, et al.,(1985); Carmichael, et al., Proc. Natl. Acad. Sci. USA 83, 2407-2411 (1986). Sequence differences between this collagenase inhibitor and others previously reported, for example by Murray, et al., J. Biol. Chem. 261, 4154-4159 (1986), may be ascribed to species variations and/or variable forms of this protein in the same tissue, although CDI appears as a single band of apparent Mr of 27,650 as determined by SDS-PAGE analysis followed by silver staining.

The N-terminal sequence of CDI is shown in Table II.

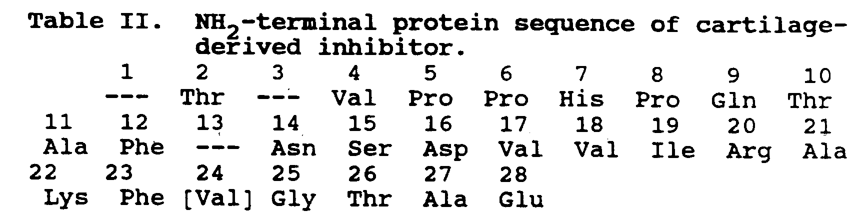

Other collagenase inhibitors can also be used. It is possible to use a material as simple as a peptide complementary to and binding to the collagenase active site, or which inhibits utilization of divalent ions by the collagenase, an antibody which blocks the collagenase activity and compounds which inhibit activation of the precursor collagenase to active collagenase, either directly or by inhibition of the enzymes required for activation of collagenase. By virtue of their specificity, these compounds are non-cytotoxic. Other proteins include platelet factor 4 and tissue inhibitor metalloproteinase (TIMP), both of which are collagenase inhibitors and which might also contain a peptide sequence which has anti-collagenase and anti-angiogenic activity.

The following demonstrates the discovery of the additional activities characterizing the CDI, and the effective dosage of CDI to inhibit angiogenesis, as compared to inhibition of collagenase activity in vitro. The CDI is purified from cartilage as described above.

### Example 1: Inhibition of Capillary Endothelial Cell Proliferation by CDI.

The first in vitro studies conducted with CDI focused on the effects of this factor on one of the important components of angiogenesis, capillary endothelial cell (BCE) proliferation.

### A. Inhibition of Endothelial Cell Proliferation measured by colorimetric determination of cellular acid phosphatase activity and electronic cell counting.

The colorimetric determination of cellular acid phosphatase activity, described by Connolly, et al., J. Anal. biochem. 152, 136-140 (1986), is used to quickly and sensitively screen small amounts of fractions from entire columns in the purification scheme for inhibition of capillary endothelial cell proliferation, as measured by color.

Screening each successive column step using this assay, it was determined that the two distinct biological activities, anti-collagenase and anti-proliferative activities, copurify throughout the purification scheme. The same results were also obtained using a second assay to study BCE proliferation based on electronic cell counting. In these studies, fractions enriched in collagenase inhibitory activity from each chromatographic step throughout the purification scheme were tested for ability to inhibit acidic fibroblast growth factor (aFGF) stimulation of BCE proliferation. CDI was found to inhibit this proliferation in a significant, dose-dependent manner. Purified CDI inhibits aFGF-stimulated capillary EC proliferation with an IC₅₀ (concentration of inhibitor at which 50% inhibition of proliferation is obtained) of 50 to 75 nM. A comparison of the IC₅₀ values (the inhibitory concentration at which 50% inhibition of growth factor-induced stimulation is observed) of CDI from the four successive steps as determined by cell counting assays reflects an increase in potency of its anti-proliferative activity as the collagenase inhibitor is purified. IC₅₀ values: A-1.5m activity: 14 µg/ml, Bio-Rex 70 activity: 9 µg/ml, Sephadex G-75 activity: 4 µg/ml, Reverse-phase HPLC activity: 0.8 µg/ml.

### B. Inhibition of Endothelial Cell Proliferation measured by DNA synthesis.

In studies to determine the effect of CDI on capillary EC synthesis of DNA, CDI suppressed the incorporation of radioactive thymidine by capillary EC in response to aFGF in a dose responsive manner with an IC₅₀ of approximately 300 nM.

### C. Comparison with CDI Inhibition of Endothelial Cell Proliferation with control.

To examine the specificity of the inhibitor's antiproliferative effect, a number of other substances, of particular relevance as control factors, were tested for their ability to inhibit capillary EC proliferation. Other enzyme inhibitors such as trypsin ovoinhibitor, pancreatic trypsin inhibitor and α₂-macroglobulin, a relatively non-specific collagenase inhibitor, as well as chondroitin sulfate A, a glycosaminoglycan found in cartilage, did not have a significant effect (less than 20% inhibition or stimulation, at all doses tested) on growth factor-stimulated capillary EC proliferation even when tested at concentrations of 50 µg/ml. These factors also do not inhibit angiogenesis in vivo. In addition, although the inhibitor inhibits aortic endothelial cells (AE), with an effective concentration that is much greater (IC₅₀ = 40 µg/ml) than for capillary endothelial cells (IC₅₀ = 14 µg/ml), the inhibitor has no detectable inhibitory effect on the growth of non-endothelial cell types, such as bovine aortic smooth muscle cells and Balb/c 3T3 cells.

### Example 2: Inhibition of Capillary Endothelial Cell Migration by CDI.

Another important component of the angiogenic process is the migration of capillary EC in response to an angiogenic stimulus, as reviewed by Ausprunk, et al., J. Microvasc. Res. 14, 53-65 (1977).

The inhibitor of EC migration copurified with the collagenase inhibitor throughout the purification. IC₅₀ values were 143, 38 and 8 µg/ml for CDI obtained at the A-1.5m, Bio-Rex 70 and Sephadex G-75 chromatography steps respectively. Purified CDI inhibits capillary EC migration with an IC₅₀ of 96 nM.

### Example 3: Inhibition in vivo of Neovascularization by CDI.

The chick chorioallantoic membrane assay (CAM), described by Taylor and Folkman, Nature (London) 297, 307-312 (1982), was used as a third bioassay to determine whether CDI was capable of inhibiting neovascularization in vivo. The effect of CDI on growing embryonic vessels was studied using chick embryos in which capillaries appear in the yolk sac at 48 h and grow rapidly over the next 6-8 days.

On day 3 of development, fertilized chick embryos were removed from their shells and placed in plastic petri dishes (1005, Falcon). They were maintained in humidified 5% CO₂ at 37°C. On day 6, 4 µg samples of purified CDI were mixed in methylcellulose disks and applied to the surfaces of the growing CAMs above the dense subectodermal plexus. Following a 48 hour exposure of the CAMs to CDI, avascular zones, free of capillaries and small vessels were observed using a binocular dissecting microscope at x7-10 magnification. The CAMs were injected intravascularly with India ink/Liposyn as described previously by Taylor and Folkman (1982).

The results show significant inhibition, as evidenced by a large avascular zone, caused by 4 µg of purified CDI (14.5 µM) applied locally in methylcellulose discs on the normally developing chorioallantoic membrane within 48 hours of exposure to inhibitor. In contrast, control CAMs implanted with empty methylcellulose disks never develop avascular zones. Histological studies of CDI-treated CAMs reveal a mesoderm which is thinner than normal and avascular relative to controls which show normal vascular development.

The centers of the avascular zones were completely free of India-ink filled capillaries; the centers of the zones contain the methylcellulose disks. This effect was observed in 33% of the eggs tested in at least two separate sets of CAM assays using several different batches of cartilage starting material. Histological sections of day 8 CAMs (x 800) reveal normal capillary development in untreated controls and controls containing an empty methylcellulose disk. In contrast, capillaries were absent in test CAMs treated with 4 µg of CDI. The anti-angiogenic activity of CDI was monitored throughout its purification using this CAM assay and dose dilution curves were generated for the inhibitor from each successive purification step through homogeneity. Purified CDI causes 100% avascular zones in the CAM at 4 µg; 25 µg of CDI from the G-75 chromatography step causes 100% avascular zones on the CAM; 50 µg of CDI from the Bio-Rex 70 column causes 80% avascular Zones, and CDI from the A-1.5 m step shows no inhibition of vascularization on the CAM.

The cartilage-derived inhibitor is a potent inhibitor of angiogenesis since inhibition was observed with as little as 4 µg. For comparison, the lowest reported doses for previously described angiogenesis inhibitors tested alone in the CAM assay are 50 µg of protamine (which is also cytotoxic at this concentration), as reported by Taylor and Folkman (1982), 200 µg of bovine vitreous extract, as reported by Lutty, et al., Invest. Opthalmol. Vis. Sci. 24, 53-56 (1983), and 10 µg of platelet factor 4, as reported by Taylor and Folkman (1982). By comparison, the cartilage-derived inhibitor is an extremely potent inhibitor of angiogenesis in vivo. The lowest reported doses of angiogenesis inhibitors effective as combinations include heparin (50 µg) and hydrocortisone (60 µg), and B-cyclodextrin tetradecasulfate (14 µg) and hydrocortisone (60 µg), reported by Eisenstein, et al., Am. J. Pathol. 81, 1-9 (1987).

### Example 4: Inhibition of Capillary Tube Formation by CDI.

Using the assay for directed migration of capillary endothelial cells from cultured BCE cells into collagen gels and determining if capillary-like tubes are formed by cultured BCE cells, CDI was demonstrated to be an effective inhibitor of angiogenesis. In control collagen gels, not treated with CDI, the monolayer of BCE cells appears as a network of branching, anastomosing cords of endothelial cells resembling capillary beds in vivo. In contrast, collagen gels treated with CDI show major disruption of the BCE cells reorganization such that any organization into capillary-like tubes appears to be prevented.

### Example 5: Inhibition of tumor-induced angiogenesis using the rabbit corneal pocket assay.

CDI, purified from bovine scapular cartilage or conditioned media of scapular chondrocytes, was shown in the foregoing examples to inhibit growth-factor stimulated proliferation and migration of capillary endothelial cell proliferation in vitro, when tested at nanomolar concentrations. Since tumor growth is dependent on neovascularization, the CDI was tested for suppression of tumor-induced angiogenesis using the rabbit corneal pocket assay. Implants of V₂ carcinoma were utilized to produce the neovascular response in the normally avascular cornea. Sustained release polymer pellets of ethylene of ethylene-vinyl acetate copolymer (Elvax) were impregnated with 40 µg each of the CDI and implanted between the tumor and the limbal edge of the cornea.

The tumors induced vessels to sprout four to six days after implantation. When mean maximal vessel lengths were measured, it was found that the CDI significantly inhibited capillary blood vessel growth in the treated versus untreated eyes (45 to 65% inhibition).

### Example 6: Preparation of Pharmaceutical Compositions containing CDI as the active ingredient.

Examples 1 to 3 demonstrate that a single, tissue-derived macromolecule, CDI, is a potent inhibitor of angiogenesis. In vitro studies using this inhibitor indicate that it negatively modulates at least three key components of the angiogenic process, the proliferation and migration of capillary endothelial cells, and tube formation. The CAM studies indicate that it also inhibits angiogenesis in an in vivo model.

Similar results have been obtained from the chondrocyte-derived inhibitor, testing for inhibition of proliferation and migration of capillary endothelial cells. In comparisons of angiogenesis inhibition, the chondrocyte derived material showed more activity than the cartilage derived CDI, which is not glycosylated following isolation using the acid precipitation. The chondrocyte material is glycosylated. It is believed that materials that are naturally expressed and glycosylated have greater activity than those materials which have been deglycosylated or expressed from recombinant sequences. However, all can be delivered in an appropriate pharmaceutical vehicle to pharmaceutical use.

Pharmaceutical compositions are prepared using CDI as the active agent to inhibit angiogenesis based on the specific application. Application is either topical or localized. For topical application, the purified CDI is combined with a carrier so that an effective dosage is delivered, based on the desired activity (i.e. ranging from an effective dosage, for example, of 14.5 µM or 4 µg to prevent localized angiogenesis and for inhibition of capillary endothelial cell migration to 100 µM for inhibition of capillary endothelial cell proliferation. A topical CDI composition is applied to the skin for treatment of diseases such as psoriasis. The carrier may be in the form of an ointment, cream, gel, paste, foam, aerosol, suppository, pad or gelled stick.

A topical CDI composition for treatment of some of the eye disorders discussed above consists of an effective amount of CDI in an acceptable ophthalmic excipient such as buffered saline, mineral oil, vegetable oils such as corn or arachis oil, petroleum jelly, Miglyol 182, alcohol solutions, or liposomes or liposome-like products. Any of these compositions may also include preservatives, antioxidants, antibiotics, immunosuppressants, and other biologically or pharmaceutically effective agents which do not exert a detrimental effect on the CDI.

CDI compositions for local or regional administration, for example, into a tumor, will generally include an inert diluent. Solutions or suspensions used for parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation can be enclosed in ampoules, disposable syringes or multiple dose vials made of glass or plastic.

For directed internal topical applications, for example for treatment of ulcers or hemorrhoids, or other lesions of mucosal membranes, the CDI composition may be in the form of tablets or capsules, which can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; or a glidant such as colloidal silicon dioxide. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents.

In a preferred form, the CDI is administered in combination with a biodegradable, biocompatible polymeric implant which releases the CDI over a controlled period of time at a selected site. Examples of preferred polymeric materials include polyanhydrides, polyorthoesters, polyglycolic acid, polylactic acid, polyethylene vinyl acetate, and copolymers and blends thereof. CDI can also be administered at a local site by infusion pump, for example, of the type used for delivering insulin or chemotherapy to specific organs or tumors.

## Claims (Claims for the following Contracting State(s): AT, BE, CH, DE, DK, FR, GB, IT, LI, LU, NL, SE)

1. An isolated and purified protein wherein the protein:
a) is a normal component of cartilage;
b) has an apparent molecular weight by SDS-PAGE under reducing conditions of about 34 kD when isolated without exposure to acid pH or of about 28 kD after exposure to pH 2.0;
c) is a collagenase inhibitor; and
d) produces an avascular zone in a chorioallantoic membrane assay when used in the amount of about 4 µg.

2. A pharmaceutical composition comprising the isolated and purified protein of Claim 1 and a pharmaceutically acceptable carrier.

3. A protein according to Claim 1 or a composition according to Claim 2 for use in medicine.

4. The use of a protein according to Claim 1 or a composition according to Claim 2 in the preparation of a medicament for the inhibition of neovascularization.

## Claims (Claims for the following Contracting State(s): ES, GR)

1. A process for the preparation of an isolated and purified protein wherein the protein:
a) is a normal component of cartilage;
b) has an apparent molecular weight by SDS-PAGE under reducing conditions of about 34 kD when isolated without exposure to acid pH or of about 28 kD after exposure to pH 2.0;
c) is a collagenase inhibitor; and
d) produces an avascular zone in a chorioallantoic membrane assay when used in the amount of about 4 µg,
the process comprising extraction from cartilage, followed by precipitation and purification by chromatographic techniques, or expression from a nucleotide sequence encoding the protein.

2. A process for the preparation of a pharmaceutical composition comprising admixing the protein prepared by the process of Claim 1 and a pharmaceutically acceptable carrier.

3. The use of a protein prepared according to Claim 1 or a composition prepared according to Claim 2 in the preparation of a medicament for the inhibition of neovascularization.

## Patentansprüche (Patentansprüche für folgende(n) Vertragsstaat(en): AT, BE, CH, DE, DK, FR, GB, IT, LI, LU, NL, SE)

1. Isoliertes und gereinigtes Protein, wobei das Protein:
a) eine normale Komponente eines Knorpels ist,
b) ein scheinbares Molekulargewicht gemäß SDS-PAGE unter reduzierenden Bedingungen von etwa 34 kD hat, wenn es isoliert wird, ohne einem sauren pH ausgesetzt zu werden, oder von etwa 28 kD, nachdem es einem pH von 2,0 ausgesetzt worden ist,
c) ein Kollagenaseinhibitor ist, und
d) eine avaskuläre Zone in einer Chorioallantoismembranprobe erzeugt, wenn es in der Menge von etwa 4 µg verwendet wird.

2. Pharmazeutische Zusammensetzung, welche das isolierte und gereinigte Protein von Anspruch 1 und einen pharmazeutisch annehmbaren Träger umfaßt.

3. Protein nach Anspruch 1 oder Zusammensetzung nach Anspruch 2 zur Verwendung in der Medizin.

4. Verwendung eines Proteins nach Anspruch 1 oder einer Zusammensetzung nach Anspruch 2 bei der Herstellung eines Medikaments zur Hemmung der Neovaskularisation.

## Patentansprüche (Patentansprüche für folgende(n) Vertragsstaat(en): ES, GR)

1. Verfahren zur Herstellung eines isolierten und gereinigten Proteins, wobei das Protein:
a) eine normale Komponente eines Knorpels ist,
b) ein scheinbares Molekulargewicht gemäß SDS-PAGE unter reduzierenden Bedingungen von etwa 34 kD hat, wenn es isoliert wird, ohne einem sauren pH ausgesetzt zu werden, oder von etwa 28 kD, nachdem es einem pH von 2,0 ausgesetzt worden ist,
c) ein Kollagenaseinhibitor ist, und
d) eine avaskuläre Zone in einer Chorioallantoismembranprobe erzeugt, wenn es in der Menge von etwa 4 µg verwendet wird,
wobei das Verfahren eine Extraktion aus dem Knorpel umfaßt, gefolgt von einer Fällung und Reinigung mit chromatographischen Techniken, oder eine Expression aus einer Nukleotidsequenz, welche das Protein kodiert.

2. Verfahren zur Herstellung einer pharmazeutischen Zusammensetzung, welches das Beimengen des durch das Verfahren nach Anspruch 1 hergestellten Proteins und eines pharmazeutisch annehmbaren Trägers umfaßt.

3. Verwendung eines nach Anspruch 1 hergestellten Proteins oder einer nach Anspruch 2 hergestellten Zusammensetzung zur Herstellung eines Medikaments zur Hemmung der Neovaskularisation.

## Revendications (Revendications pour l'(les) Etat(s) contractant(s) suivant(s): AT, BE, CH, DE, DK, FR, GB, IT, LI, LU, NL, SE)

1. Protéine isolée et purifiée où la protéine :
a) est un composant normal du cartilage;
b) a un poids moléculaire apparent par SDS-PAGE en conditions réductrices de 34 kD quand elle est isolée sans exposition à pH acide ou d'environ 28 kD après exposition à pH 2,0;
c) est un inhibiteur de la collagénase; et
d) produit une zone avasculaire dans un essai sur membrane chorioallantoïque lors d'une utilisation à la quantité d'environ 4 µg.

2. Composition pharmaceutique comprenant la protéine isolée et purifiée de la revendication 1 et un support pharmaceutiquement acceptable.

3. Protéine selon la revendication 1 ou une composition selon la revendication 2 pour une utilisation en médecine.

4. Utilisation d'une protéine selon la revendication 1 ou d'une composition selon la revendication 2 dans la préparation d'un médicament pour l'inhibition de la néovascularisation.

## Revendications (Revendications pour l'(les) Etat(s) contractant(s) suivant(s): ES, GR)

1. Procédé de préparation d'une protéine isolée et purifiée où la protéine :
a) est un composant normal du cartilage;
b) a un poids moléculaire apparent par SDS-PAGE en conditions réductrices de 34 kD quand elle est isolée sans exposition à un pH acide ou d'environ 28 kD après exposition à pH 2,0;
c) est un inhibiteur de la collagénase; et
d) produit une zone avasculaire dans un essai sur membrane chorioallantoïque lors d'une utilisation en la quantité d'environ 4 µg.
le procédé comprenant l'extraction du cartilage avec ensuite précipitation et purification par des techniques chromatographiques ou bien l'expression d'une séquence de nucléotides codant pour la protéine.

2. Procédé pour la préparation d'une composition pharmaceutique consistant à mélanger la protéine préparée par le procédé de la revendication 1 et un support pharmaceutiquement acceptable.

3. Utilisation d'une protéine selon la revendication 1 ou d'une composition préparée dans la revendication 2 dans la préparation d'un médicament pour l'inhibition de la néovascularisation.
